# EUROPEAN PATENT SPECIFICATION

(11) **EP 1 013 724 B1**
(45) Date of publication and mention of the grant of the patent: **09.07.2003**
(21) Application number: 99125928.4
(22) Date of filing: 23.12.1999
(51) Int. Cl.: C09C 1/00, C09D 11/02

(54) **Pigment mixture**
Pigment-Mischung
Mélange de pigments

(30) Priority: 23.12.1998 EP 98124473
(43) Date of publication of application: 28.06.2000
(73) Proprietor: MERCK PATENT GmbH, 64293 Darmstadt (DE)
(72) Inventor: Pfaff, Gerhard, Dr., 64839 Münster (DE); Schoen, Sabine, Dr., 64287 Darmstadt (DE); Nishimagi, Atsuko, Takijiri, Fukushima-pref, 971-8182 (JP)

(56) References cited:
- EP-A- 0 763 573
- DE-A- 19 820 112

## Description

The present invention relates to pigment mixtures containing at least two components, component A being Al₂O₃ flakes coated with one or more metals, metal oxides and/or metal sulfides and component B being special-effect pigments, and to their use in varnishes, paints, printing inks, plastics and cosmetic formulations.

With platelet-shaped pigments, hiding power and gloss are often difficult to realise simultaneously to a satisfactory extent. For instance, SiO₂ flakes or mica platelets covered with one or more thin metal oxide layers feature interference colours and a high lustre but at the same time, owing to the transparent substrate, feature high transparency and hence a comparatively poor hiding power.

Thus EP 0 562 329 discloses a pigment mixture comprising iron oxide-coated SiO₂ flakes in combination with iron oxide-coated mica pigments.

DE-A-42 40 511 discloses a pigment mixture which consists of an interference pigment and a platelet-shaped colour pigment. The interference pigment comprises metal oxide-coated mica flakes or SiO₂ flakes, and the colour pigment can be coloured, uncoated SiO₂ flakes. This pigment mixture is incorporated into coating materials, printing inks or plastics.

The object of the present invention is to provide a pigment mixture which is notable for relatively high hiding power and which tends itself well to incorporation into the respective system in which it is used, and for which at the same time a separation of pigment/colourant in the system is largely ruled out.

Surprisingly, a pigment mixture has now been found which has none of the disadvantages indicated above. The pigment mixture of the invention consists of at least two components, component A being Al₂O₃ flakes coated with one or more metals, metal oxides and/or metal sulfides and component B being one or more special-effect pigments. The admixture of the special-effect pigments to the coated Al₂O₃ flakes is able to give the systems in which they are used a multiple flop, the colour effect is intensified, and new colour effects are achieved.

The invention thus provides a pigment mixture containing at least two components, component A being Al₂O₃ flakes coated with one or more metals, metal oxides and/or metal sulfides and component B being one or more special-effect pigments.

The invention likewise provides the formulations, such as paints, varnishes, printing inks, plastics, agricultural films and cosmetic formulations, which comprise the pigment mixture of the invention.

The coated Al₂O₃ flakes can be mixed in any proportion with the special-effect pigments. The ratio of component A to component B is preferably from 1:10 to 10:1, in particular from 3:1 to 5:1.

Coated aluminum oxide in a flaky form is commercial available for example from Merck KGaA under the tradename Xirallic®.

α-Al₂O₃ in the form of hexagonal flakes having a particle diameter greater than 10 µm and an aspect ratio (particle diameter/thickness) of 5-10 is known from JP 111239/1982 (Laid Open No.).

The Japanese Patent Publication No. 72527/1991 discloses α-Al₂O₃ in the form of flakes having an average particle diameter of 0,5-3 µm.

The JP 39362/1992 (Laid Open No.) describes Al₂O₃ in the form of fine platy particles of a hexagonal crystal system with the plane perpendicular to the c axis grown into a plate.

Preferred Al₂O₃ flakes are flakes composed of aluminum oxide (as a major constituent) and of titanium dioxide (as a minor constituent) which are known from U.S. 5,702,519. These Al₂O₃ flakes are prepared from a uniform aqueous solution water-soluble aluminum salt and titanium salt by hydrolysis with an alkali carbonate aqueous solution in the presence of an aqueous solution containing an alkali metal salt like alkali metal sulfate and phosphoric acid or phosphate, drying by evaporation (dehydration by heating), and molten salt treatment.

The Al₂O₃ flakes are provided with one or more metal oxide layers. Examples of suitable metal oxides or metal oxide mixtures are titanium dioxide, zirconium oxide, zinc oxide, iron oxides (Fe₂O₃ and/or Fe₃O₄) and/or chromium oxide, especially TiO₂ and/or Fe₂O₃. The Al₂O₃ flakes can be coated in the same way as pearl lustre pigments. Coatings with a metal oxide may be accomplished by any known methods, such as hydrolysis of a metal salt by heating or alkali, which deposits hydrated metal oxide, followed by calcination.

Al₂O₃ flakes can also be coated with one or more layers of a metal or metal alloy selected e.g. from chromium, nickel, silver, bismuth, copper, tin, or hastalloy. Al₂O₃ flakes coated with a metal sulfide are coated with sulfides e.g. of tungsten, molybdenum, cerium, lanthanum or rare earth elements.

The Al₂O₃ flakes can be coated by wet chemical coating, by CVD or PVD processes.

Suitable components B for the pigment mixture of the invention are all special-effect pigments familiar to the skilled worker in the effect pigment sector, examples being metal effect pigments, such as aluminum, copper, zinc, tin and their alloys. Aluminum and gold bronze alloys are preferably to be mentioned, especially those having a particle size of 2 to 40 µm.

The pigment mixtures of the invention preferably comprise uncoated and coated platelet-shaped iron oxide, aluminum flakes or coated aluminum flakes. Special-effect pigments of this kind are marketed by BASF under the name Paliocrom®, by Eckart-Werke under the name Stapa®, and by the Flex Company under the name ChromaFlair®. The following pigments should also be mentioned: Pearl lustre pigments, TiO₂ flakes or SiO₂ flakes coated with one or more metal oxides, such as TiO₂ or Fe₂O₃, for example, graphite platelets, BiOCl or glass flakes coated with one or more metal oxides, liquid crystal polymer particles (LCP), holographic pigments and multilayer pigments.

Pearl lustre pigments, mica flake pigments coated with one or more metal oxides, are obtainable, for example, from Merck KGaA, Darmstadt, under the tradenames Iriodin®, Afflair® and Timiron®. The latter pigments are known, for example, from the German Patents and Patent Applications 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 and 32 53 017. Mica pigments coated with TiO₂ and/or Fe₂O₃ are employed in particular. As phyllosilicate both natural and synthetic mica are suitable.

Additionally, the inventive pigment mixture can contain organic or inorganic colourants, thixotropic agents, wetting agents, dispersing agents, water, organic solvent or solvent mixtures, etc.

The pigment mixture of the invention is simple and easy to handle. The pigment mixture can be incorporated into the system in which it is used by simple stirring. Laborious milling and dispersing of the pigments is not necessary.

The pigment mixture of the invention can be used for pigmenting coating materials, printing inks, plastics, agricultural films, button pastes, for the coating of seed, for the colouring of food, coatings of medicaments or cosmetic formulations. The concentration of the pigment mixture in the system in which it is to be used for pigmenting is generally between 0.01 and 50 % by weight, preferably between 0.1 and 5 % by weight, based on the overall solids content of the system. This concentration is generally dependent on the specific application.

Plastics comprising the pigment mixture of the invention in amounts of 0.1 to 50 % by weight, in particular from 0.5 to 7 % by weight, are frequently notable for a particular sparkle effect.

In the coating sector, especially in automotive finishing, the pigment mixture is employed in amounts of 0.5-10 % by weight. The proportion in which the coated Al₂O₃ flakes are mixed with component B, especially coated SiO₂ flakes or coated mica platelets, depends on the desired effect. The Al₂O₃ flakes are preferably employed with component B in a ratio of 1:10 to 10:1, in particular of 3:1.

In the coating material, the pigment mixture of the invention has the advantage that the desired colour flop effect is obtained by a single-layer coating (one-coat systems or as a base coat in a two-coat system).This colour flop is extremely pronounced even under diffuse light. In comparison with coatings which comprise an interference pigment rather than the coated Al₂O₃ flakes, coatings with the pigment mixture of the invention exhibit a marked depth effect and a glitter effect and also a strong colour flop.

In the pigmentation of binder systems, for example for paints and printing inks for intaglio, offset or screen printing, pigment mixtures consisting of coated Al₂O₃ flakes with Stapa®-aluminum and gold bronze pastes from Eckart-Werke have proven particularly suitable. The pigment mixture is incorporated into the printing ink in amounts of 2-50 % by weight, preferably 5-30 % by weight and, in particular, 8-15 % by weight. The mixing ratio of component A to component B is preferably in the range from 1:10 to 10:1. The printing inks comprising the pigment mixture of the invention exhibit purer hues and are of improved printability owing to the good viscosity values.

The invention likewise provides pigment preparations comprising coated Al₂O₃ flakes, effect pigments, binders and, if desired, additives, the said preparations being in the form of substantially solvent-free, free-flowing granules. Such granules contain up to 95 % by weight of the pigment mixture. A pigment preparation in which the pigment mixture of the invention is pasted up with a binder and with water and/or an organic solvent, with or without additives, and the paste is subsequently dried and brought into a compact particulate form, e.g. granules, pellets, briquettes, a masterbatch or tablets, is particularly suitable as a precursor for printing inks.

The present invention therefore also provides formulations containing the pigment mixture of the invention.

The examples which follow are intended to illustrate the invention without, however, limiting it.

### Examples

### Example 1: Paint

Formulations consisting of

| | |
|---|---|
| 2.50 % | Fe₂O₃-coated Al₂O₃ flakes having a particle size of 5-60 µm (Merck KGaA) |
| 1.50 % | Monastral green 6Y spec. (Zeneca) |
| 0.50 % | Cappoxyt yellow 4214 (Capelle) |
| 0.03 % | Pigment-grade carbon black FW 200 (Degussa) |
| 0.40 % | Dollaraluminum Alpate 7620 NS (Alcan Toyo Europe) |

- Remainder:: Paint base with 19 % solids content (acrylate-melamine) and diluent mixture

### Example 2: Intaglio printing

Printing ink consisting of

| | |
|---|---|
| 70 g | nitrocellulose-based binder from Gebrüder Schmidt, 95MB011, with solids content of about 20 % |
| 30 g | pigment, i.e. 15 g of Cromal IV (Eckart) AL 14-18 µm and 15 g of Fe₂O₃-coated Al₂O₃ flakes of particle size 5-60 µm |
| 30 g | 1-ethoxy-2-propanol |

### Example 3: Plastic

1 kg of polystyrene granules are wetted uniformly in a tumble mixer with 5 g of adhesion agent. Then 35 g of Fe₂O₃-coated Al₂O₃ flakes of particle size 5-60 µm and 7g of Iriodin® 121 (TiO₂-coated mica pigment from Merck KGaA, Darmstadt, FRG with particle size 5-20 µm) are added and the components are mixed for 2 minutes.

These granules are processed under customary conditions on an injection moulding machine to give small stepped plates measuring 4 x 3 x 0.5 cm. The small stepped plates are notable for their lustre.

### Example 4: Eye shadow

### Phase A

| | |
|---|---|
| 15.00 % | TiO₂-coated Al₂O₃ flakes of particle size 5-60 µm (Merck KGaA) |
| 15.00 % | Timiron® Super Blue (TiO₂-coated mica of particle size 10-60 µm from Merck KGaA) |
| 49.50 % | Talc |
| 7.50 % | Solarium Tuberosum (potato starch) |
| 2.50 % | Magnesium stearate |

### Phase B

| | |
|---|---|
| 9.14 % | Isopropyl stearate |
| 0.53 % | Cetyl palmitate |
| 0.53 % | Petrolatum |
| 0.21 % | Fragance |
| 0.11 % | Preservative |

The constituents of Phase A are combined and formed into a premix. The melted phase B is then added dropwise with stirring to the powder mixture. The powders are pressed at 40-50 bar.

### Example 5: Shower gel

### Phase A

| | |
|---|---|
| 0.10 % | TiO₂-coated Al₂O₃ flakes of particle size 5-60 µm (Merck KGaA) |
| 0.10 % | Timiron® Super Blue (TiO₂-coated mica of particle size 10-60 µm from Merck KGaA) |
| 0.75 % | Xantham gum |
| ad 100.00 % | Aqua |

### Phase B

| | |
|---|---|
| 20.00 % | Decyl glycoside |
| 6.65 % | Texapon ASV Sodium laureth sulfate Magnesium laureth sulfate Sodium laureth 8-sulfate Magnesium laureth 8-sulfate Sodium oleth sulfate |
| 0.20 % | Preservative |
| 0.50 % | Fragrance |

### Phase C

| | |
|---|---|
| 0.15% | Citric acid |
| 10.00% | Aqua |

For Phase A, the pigments are stirred into the water. The xanthan gum is scattered in slowly with stirring and the mixture is stirred until the gum has dissolved. Phases B and C are added in succession, and slow stirring is continued until all of the components are homogeneously distributed.

## Claims

1. Pigment mixture containing at least two components, component A being Al₂O₃ flakes coated with one or more metals, metal oxides and/or metal sulfides and component B being special-effect pigments selected from the group of metal platelets coated with one or more metal oxides, graphite platelets, aluminum platelets, phyllosilicates, Fe₂O₃-, SiO₂- or TiO₂-flakes uncoated or coated with one or more metal oxides, glass platelets and/or ceramic platelets.

2. Pigment mixture according to Claim 1, **characterised in that** component A comprises TiO₂- and/or Fe₂O₃- coated Al₂O₃ flakes.

3. Pigment mixture according to one of Claims 1 to 2, **characterised in that** component A and component B are mixed in a ratio of 10:1 to 1:10.

4. Use of the pigment mixture according to Claim 1 in paints, varnishes, printing inks, powder coating materials, master batches, plastics, for colouring seed, in cosmetic formulations and for enhancing foods.

5. Formulations containing a pigment mixture according to Claim 1.

6. Pigment preparation containing a pigment mixture according to Claim 1 and a binder, **characterised in that** it is in the form of substantially solvent-free, free-flowing granules.

## Patentansprüche

1. Pigmentgemisch enthaltend mindestens zwei Komponenten, wobei Komponente A mit einem oder mehreren Metallen, Metalloxiden und/oder Metallsulfiden beschichtete Al₂O₃-Flakes und Komponente B Effektpigmente ausgewählt aus der Gruppe der mit einem oder mehreren Metalloxiden beschichteten Metallplättchen, Graphitplättchen, Aluminiumplättchen, Schichtsilikate, unbeschichteten oder mit einem oder mehreren Metalloxiden beschichteten Fe₂O₃-, SiO₂- oder TiO₂-Flakes, Glasplättchen und/oder Keramikplättchen sind.

2. Pigmentgemisch nach Anspruch 1, **dadurch gekennzeichnet, dass** Komponente A mit TiO₂ und/oder Fe₂O₃ beschichtete Al₂O₃-Flakes enthält.

3. Pigmentgemisch nach einem der Ansprüche 1 bis 2, **dadurch gekennzeichnet, dass** Komponente A und Komponente B im Verhältnis 10:1 bis 1:10 gemischt sind.

4. Verwendung des Pigmentgemisches nach Anspruch 1 in Farben, Lacken, Druckfarben, Pulverlacken, Masterbatches, Kunststoffen, zur Saatguteinfärbung, in kosmetischen Formulierungen und zur Veredelung von Lebensmitteln.

5. Formulierungen enthaltend ein Pigmentgemisch nach Anspruch 1.

6. Pigmentzubereitung enthaltend ein Pigmentgemisch nach Anspruch 1 und ein Bindemittel, **dadurch gekennzeichnet, dass** sie in Form eines weitgehend lösungsmittelfreien, rieselfähigen Granulats vorliegt.

## Revendications

1. Mélange de pigments contenant au moins deux composants, le composant A étant des paillettes d'Al₂O₃ revêtues d'un ou plusieurs métaux, oxydes métalliques et/ou sulfures métalliques et le composant B étant des pigments à effets spéciaux choisis dans le groupe constitué par des lamelles métalliques revêtues d'un ou plusieurs oxydes métalliques, des lamelles de graphite, des lamelles d'aluminium, des phyllosilicates, des paillettes de Fe₂O₃, SiO₂ ou TiO₂ non revêtues ou revêtues d'un ou plusieurs oxydes métalliques, des lamelles de verre et/ou des lamelles de céramique.

2. Mélange de pigments selon la revendication 1, **caractérisé en ce que** le composant A comprend des paillettes d'Al₂O₃ revêtues de TiO₂ et/ou de Fe₂O₃.

3. Mélange de pigments selon la revendication 1 ou 2, **caractérisé en ce que** le composant A et le composant B sont mélangés dans un rapport compris entre 10:1 et 1:10.

4. Utilisation du mélange de pigments selon la revendication 1 dans les peintures, vernis, encres d'imprimerie, matériaux de revêtements de poudre, mélange-maîtres, matières plastiques, pour la coloration des semences, dans les formulations cosmétiques et pour la mise en valeur d'aliments.

5. Formulations contenant un mélange de pigments selon la revendication 1.

6. Préparation pigmentaire contenant un mélange de pigments selon la revendication 1 et un liant, **caractérisée en ce qu'**elle est sous la forme de granulés essentiellement sans solvants, qui s'écoulent librement.
